# FASCICULE DE BREVET EUROPEEN

(11) **EP 1 239 861 B1**
(45) Date de publication et mention de la délivrance du brevet: **04.04.2007**
(21) Numéro de dépôt: 00988905.6
(22) Date de dépôt: 08.12.2000
(51) Int. Cl.: A61K 31/55, A61K 31/54, A61P 25/00

(54) **ASSOCIATION DE CYAMEMAZINE ET D'UN NEUROLEPTIQUE ATYPIQUE**
KOMBINATION ENTHALTEND CYAMEMAZINE UND EIN ATYPISCHES NEUROLEPTIKUM
COMBINATION OF CYAMEMAZINE AND AN ATYPICAL NEUROLEPTIC

(30) Priorité: 10.12.1999 FR 9915632
(43) Date de publication de la demande: 18.09.2002
(73) Titulaire: Aventis Pharma S.A., 92160 Antony (FR)
(72) Inventeur: DIB, Michel, F-75013 Paris (FR); LEPERLIER, Cyrille, F-77300 Fontainebleau (FR)
(74) Mandataire: Rousseau, Pierrick Edouard
(86) Numéro de dépôt international: PCT/FR2000/003446
(87) Numéro de publication internationale: WO 2001/041769

(56) Documents cités:
- WO-A-98/43646
- FR-A- 2 722 099
- ZINI R ET AL: "THE INFLUENCE OF VARIOUS DRUGS ON THE BINDING OF TIANEPTINE TO HUMAN PLASMA PROTEINS." INT J CLIN PHARMACOL THER TOXICOL, (1991) 29 (2), 64-66., XP000912070
- SENNINGER J.-L. ET AL: "LA RISPERIDONE DANS LE TRAITEMENT DE L'AGRESSIVITE DES SCHIZOPHRENES." ANNALES MEDICO-PSYCHOLOGIQUES, (1998) 156/3 (210-213)., XP000912076

## Description

La présente invention concerne l'association de cyamémazine et d'un neuroleptique atypique choisi parmi l'olanzapine, la rispéridone, le sertindole, la quétiapine ou la ziprazidone, ou un de leurs sels pharmaceutiquement acceptables et son utilisation dans le traitement de la schizophrénie et, notamment des épisodes aigus de la schizophrénie.

La cyamémazine ou cyamépromazine (TERCIAN^{R}) sous forme de base ou d'un sel pharmaceutiquement acceptable et notamment son tartrate est un neuroleptique de type phénothiazine (US2877224), utilisé dans le traitement symptomatique de l'anxiété sous toutes ses formes et pouvant être éventuellement associé à un antidépresseur dans les dépressions graves.

Les neuroleptiques atypiques appelés aussi antipsychotiques atypiques sont des neuroleptiques qui produisent peu ou pas d'effets secondaires extrapyramidaux.

Parmi les neuroleptiques atypiques commercialisés, on peut citer les produits suivants :
la rispéridone (RISPERDAL^{R}) et ses sels pharmaceutiquement acceptables et notamment le pamoate (brevets EP196132, US4804663, W09425460);
l'olanzapine (ZYPREXA^{R}) et ses sels pharmaceutiquement acceptables (EP454436, US5229382); et notamment le pamoate (W09916313), ses formes polymorphes et notamment la forme II (WO9630375) et ses formes solvatées (EP733634, EP831097).

D'autres neuroleptiques atypiques sont en cours de développement; parmi ceux-ci on peut citer :
le sertindole et ses sels pharmaceutiquement acceptables (EP200322, EP392959);
la quétiapine et ses sels pharmaceutiquement acceptables (EP240228);
la ziprasidone et ses sels pharmaceutiquement acceptables (US4831031) et son monohydrate (EP586191).

Le document FR A2 722 099 décrit la combinaison de la carpipramine et d'un neuroleptique dans le traitement des mouvements anormaux liés à la prise de neuroleptique.

Ces produits sont actuellement recommandés dans le traitement de première intention de la schizophrénie. Leur efficacité sur la symptomatologie positive et négative ainsi que sur le profil de tolérance (absence ou moindres effets extrapyramidaux) constituent un progrès thérapeutique pour les patients.

Cependant, l'observation clinique montre que, pour de nombreux patients schizophrènes, l'instauration d'un traitement neuroleptique de classe atypique n'apporte pas de réponse clinique rapide alors qu'il s'agit d'une période particulièrement critique : angoisse avec agitation, agressivité, troubles du comportement pouvant mettre en danger le patient et les soignants.

Il a été proposé d'utiliser les benzodiazépines comme traitement sédatif associé mais leur intérêt reste limité (faible pourcentage de répondeurs, nécessité de fortes doses) et leurs effets secondaires non négligeables (sédation ou excitation paradoxale, risque de dépendance, de sevrage et d'abus) (Wolkowitz OM, Pickar D, Am. J. Psychiatry 148, 714-26 (1991) ; Dietch JT, Jennings RL, J. Clin. Psychiatry, 49, 184-88 (1988); Warneke LB, Can J. Psychiatry,36, 194-205 (1991); Glen L Stimmel, Pharmacotherapy, 16(6Pr 2),148S-151S)(1996)).

Il a maintenant été trouvé que chez ces patients, l'association d'un neuroleptique atypique choisi parmi l'olanzapine, la rispéridone, le sertindole, la quétiapine ou la ziprasidone, avec la cyamémazine permet un effet thérapeutique net et rapide sur l'anxiété, la tension, l'agressivité et l'excitation, avec une très bonne tolérance et une réduction globalement plus rapide de la symptomatologie schizophrénique. Cette synergie d'action peut favoriser l'observance des patients par l'amélioration rapide de leurs troubles.

Cet effet a été déterminé sur un groupe de 15 patients schizophrènes selon le DSM IV (JD Guelfi et al.,Masson Paris:1008p (1996)), et hospitalisés.

Dans un premier groupe contrôle, 7 patients ont été traités par un neuroleptique atypique seul : 5 patients par 10 à 20 mg/jour en 1 prise d'olanzapine et 2 patients par 4 à 8 mg/jour en 2 prises pendant 6 semaines.

Dans un deuxième groupe, 8 patients ont été traités par une association de cyamémazine et d'un neuroleptique atypique :
5 patients par 10 à 20 mg/jour d'olanzapine en 1 prise et 150 mg/jour de cyamémazine en 2 prises pendant 6 semaines,
3 patients par 4 à 8 mg/jour de rispéridone en 2 prises et 150 mg/jour de cyamémazine en 2 prises pendant 6 semaines.

Dans les groupes traités avec l'association, les patients n'ont pas eu de troubles du comportement au cours de l'hospitalisation et la moitié des patients ont présenté une réduction d'au moins 40 % du score BPRS (Brief Psychiatric Rating Scale) (Overall JE, Gorham DR, Psychol. Rep.,10:799-812 (1962)) pendant les 3 premières semaines de traitement, ce qui n'a pas été observé dans le groupe traité par un neuroleptique atypique seul.

La tolérance (signes extra-pyramidaux et akathisie) s'est révélée comparable dans tous les groupes.

Ces résultats démontrent que l'association d'un neuroleptique atypique et de la cyamémazine a un effet de synergie dans le traitement des troubles schizophréniques et présente un très grand intérêt dans la pratique clinique.

Selon l'invention, il est entendu que les associations peuvent comprendre un deuxième neuroleptique atypique.

Les neuroleptiques atypiques cités précédemment et la cyamémazine peuvent se présenter sous forme d'un sel pharmaceutiquement acceptable et notamment sous forme de sel d'addition avec les acides minéraux tels que acide chlorhydrique, acide bromhydrique, acide sulfurique, acide phosphorique, acide nitrique ou organiques tels que acide acétique, acide propionique, acide succinique, acide oxalique, acide benzoïque, acide fumarique, acide maléique, acide méthane sulfonique, acide iséthionique, acide théophilline-acétique, acide pamoïque, acide salicylique, acide phénolphtalinique, acide méthylène-bis-β-oxynaphtoïque, acide carbonique, acide citrique, acide lactique, acide palmitique ou des dérivés de substitution de ces dérivés.

Parmi ces associations, sont préférées les associations de cyamémazine et d'olanzapine, de cyamémazine et d'olanzapine sous forme de pamoate, de cyamémazine et d'olanzapine sous sa forme polymorphe II, de cyamémazine et de risperidone, de cyamémazine et de rispéridone sous forme de pamoate.

L'association peut être employée par voie orale, parentérale ou rectale soit simultanément soit séparément soit de manière étalée dans le temps.

La présente invention concerne également les compositions pharmaceutiques comprenant l'association de cyamémazine et d'un neuroleptique atypique choisi parmi l'olanzapine, la rispéridone, le sertindole, la quétiapine ou la ziprasidone, à l'état pur ou sous forme d'une association avec un ou plusieurs diluants et/ou adjuvants compatibles et pharmaceutiquement acceptables et/ou éventuellement en association avec un autre produit pharmaceutiquement compatible et physiologiquement actif.

Comme compositions solides pour administration orale, peuvent être utilisés des comprimés, des pilules, des poudres (capsules de gélatine, cachets) ou des granulés.

Dans ces compositions, les principes actifs sont mélangés à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semisynthétiques ou des polyéthylèneglycols.

La présente invention concerne également l'utilisation d'une association de cyamémazine et d'un neuroleptique atypique tel que décrit ci-dessus ou un de leurs sels pharmaceutiquement acceptables pour la préparation d'un médicament pour le traitement de la schizophrénie et, notamment des épisodes aigus de la schizophrénie. Cette utilisation peut être simultanée, séparée ou étalée dans le temps.

La présente invention concerne également la méthode de traitement d'un patient schizophrène et, notamment lors des épisodes aigus de la schizophrénie qui consiste à administrer au patient une association de cyamémazine et d'un neuroleptique atypique tel que décrit ci-dessus ou un de leurs sels pharmaceutiquement acceptables soit simultanément soit séparément soit de manière étalée dans le temps.

Les doses dépendent de l'effet recherché, de la durée du traitement et de la voie d'administration utilisée.

Elles sont généralement de 50 à 600 mg par jour de cyamémazine. Les doses de neuroleptique atypique sont généralement les doses préconisées en utilisation unique. Ainsi, la dose sera de 1 à 20 mg par jour d'olanzapine ou bien de 1 à 16 mg par jour de rispéridone ou bien de 1 à 24 mg par jour de sertindole ou bien de 1 à 800 mg par jour de quétiapine ou bien de 20 à 160 mg par jour de ziprasidone.

De préférence, la dose d'olanzapine est de 5 à 20 mg/jour ou bien la dose de rispéridone est de 4 à 8 mg/jour ou bien la dose de sertindole est de 2 à 8 mg/jour ou bien la dose de quétiapine est de 225 à 800 mg/jour ou bien la dose de ziprasidone est de 80 à 160 mg /jour.

Lorsque deux neuroleptiques atypiques sont utilisés dans l'association, les doses de chacun seront adaptées pour obtenir l'effet recherché.

D'une façon générale, le médecin déterminera la posologie appropriée en fonction de l'âge, du poids et de tous les autres facteurs propres au sujet à traiter.

## Revendications

1. - Association de cyamémazine et d'un neuroleptique atypique et, le cas échéant, d'un deuxième neuroleptique atypique, choisis parmi l'olanzapine, la rispéridone, le sertindole, la quétiapine ou la ziprasidone, ou un de leurs sels pharmaceutiquement acceptables.

2. - Association selon la revendication 1 contenant 50 à 600 mg de cyamémazine.

3. - Association selon l'une des revendications 1 et 2 comme préparation combinée pour une utilisation simultanée, séparée ou étalée dans le temps.

4. - Utilisation de l'association selon l'une des revendications 1 et 2 pour la préparation d'un médicament destiné au traitement de la schizophrénie.

5. - Utilisation de l'association selon l'une des revendications 1 et 2 pour la préparation d'un médicament utile pour le traitement des épisodes aigus de la schizophrénie.

6. - Composition pharmaceutique constituée d'une association selon l'une des revendications 1 et 2 à l'état pur ou en présence de tout diluant ou adjuvant compatible et pharmaceutiquement acceptable.

## Claims

1. Combination of cyamemazine and an atypical neuroleptic, and, if appropriate, a second atypical neuroleptic, chosen from olanzapine, risperidone, sertindole, quetiapine and ziprasidone, or a pharmaceutically acceptable salt thereof.

2. Combination according to Claim 1, containing 50 to 600 mg of cyamemazine.

3. Combination according to either of Claims 1 and 2, as a combined preparation for simultaneous use, separate use or use spread out over time.

4. Use of the combination according to either of Claims 1 and 2, for preparing a medicinal product intended for treating schizophrenia.

5. Use of the combination according to either of Claims 1 and 2, for preparing a medicinal product of use for treating acute episodes of schizophrenia.

6. Pharmaceutical composition consisting of a combination according to either of Claims 1 and 2, in the pure state or in the presence of any compatible and pharmaceutically acceptable diluent or adjuvant.

## Patentansprüche

1. Verbindung aus Cyamemazin und einem atypischen Neuroleptikum und gegebenenfalls einem zweiten atypischen Neuroleptikum, ausgewählt aus Olanzapin, Risperidon, Sertindol, Quetiapin oder Ziprasidon, oder aus einem ihrer pharmazeutisch annehmbaren Salze.

2. Verbindung nach Anspruch 1, enthaltend 50 bis 600 mg Cyamemazin.

3. Verbindung nach Anspruch 1 und 2 als Kombinationspräparat für eine gleichzeitige, getrennte oder zeitlich gestaffelte Verwendung.

4. Verwendung der Verbindung nach einem der Ansprüche 1 und 2 für die Aufbereitung eines Medikamentes für die Behandlung der Schizophrenie.

5. Verwendung der Verbindung nach einem der Ansprüche 1 und 2 für die Aufbereitung eines für die Behandlung von akuten Schüben der Schizophrenie nützlichen Medikamentes.

6. Pharmazeutische Zusammensetzung, bestehend aus einer Verbindung nach einem der Ansprüche 1 und 2 im Reinzustand oder in der Gegenwart von jedem kompatiblen und pharmazeutisch annehmbaren Verdünner oder Adjuvans.
